Europäisches Patentamt

**European Patent Office**

**Office européen des brevets**

(19)

(11) Publication number: **0 146 627**

**A1**

(12)

# EUROPEAN PATENT APPLICATION

published in accordance with Art. 158(3) EPC

(21) Application number: **83902696.0**

(22) Date of filing: **24.08.83**

Data of the international application taken as a basis

(86) International application number.
**PCT/JP83/00277**

(87) International publication number
**WO84/04108 (25.10.84 84/25)**

(51) Int. Cl⁴: **C 12 N 15/00**
**C 07 H 21/00, C 12 P 19/34**
**C 12 N 1/20, C 12 P 21/00**
**//A61K37/04, (C12N1/20,**
**C12R1:19)**

(30) Priority: **09.04.83 JP 62563/83**

(43) Date of publication of application:
**03.07.85 Bulletin 85/27**

(84) Designated Contracting States:
**DE FR GB**

(71) Applicant **KYOWA HAKKO KOGYO CO., LTD.**
**Ohtemachi Bldg., 6-1 Ohtemachi l-chome**
**Chiyoda-ku Tokyo 100(JP)**

(72) Inventor: **NABESHIMA, Yoichi**
**2320-4, Sakai Niigata-shi**
**Niigata 950-21(JP)**

(72) Inventor: **NABESHIMA, Yoko**
**2320-4, Sakai Niigata-shi**
**Niigata 950-21(JP)**

(72) Inventor: **OGATA, Kikuo**
**271, Sekiyahonson-cho 2-chome Niigata-shi**
**Niigata 951(JP)**

(74) Representative: **Vossius Vossius Tauchner Heunemann**
**Rauh**
**Siebertstrasse 4 P.O. Box 86 07 67**
**D-8000 München 86(DE)**

(54) **NOVEL IMMUNOGLOBULIN-CODING DNA.**

(57) Novel messenger RNA (mRNA) coding human immunoglobulin D (IgD) and DNA complementary to the mRNA (cDNA), a process for their preparation, and a process for preparing IgD using the mRNA or cDNA. IgD relates to maturation or differentiation of antibody-yielding B cells and autoimmune diseases such as rheumatism, and a DNA coding H-chain of IgD can be used as an agent for diagnosing diseases induced by abnormality of IgD.

Fig. 1

Specification

Novel DNA Coding for Immunoglobulin

Technical Field

This invention relates to a novel messenger RNA (referred to as "mRNA" hereinafter) coding for human immunoglobulin D (referred to as "IgD" hereinafter), a DNA complementary to the mRNA (referred to as "cDNA" hereinafter), a process for producing the mRNA and cDNA, and a process for producing IgD, using the mRNA and cDNA.

Background Art

Molecules that exist in an animal fluid and specifically combine with an antigen are called antibody, and protein having an antibody activity is generically called immunoglobulin. All the immunoglobulin molecules have a common structure consisting of 4 polypeptide chains, among which the larger two polypeptide chains are called H chains, and the smaller chains L chains. Human immunoglobulins are classified into 5 species, i.e. IgG, IgA, IgM, IgD, and IgE, on the basis of primary structures of the respective H chains, and the respective H chains are called $\gamma$, $\alpha$, $\mu$, $\delta$ and $\epsilon$. Among them, IgD has been hard to separate from human serum, because it exists only at a low concentration (3 - 30 µg/ml) in the serum, but recently an amino acid sequence in the constant region of $\delta$ chain in the human IgD isolated from tumor cells producing IgD has been determined [Lin. et al.: Proc. Natl. Acad. Sci., 78 504 - 508 (1981); Shinoda, et al.: ibid. 785 - 789; Putnam, et al.: ibid. 6168 - 6172].

IgD consists of two delta H chains and two lambda L chains, which are linked by three intrachain disulfide bonds.

Molecular weight of IgD amounts to 170,000 and molecular weight of single H chain amounts to 60,000.

Functions of IgD have not been fully clarified, but seem to relate to maturation and differentiation of B cells producing the antibody. Most of human B lymphocytes have both IgD and monomer IgM on their cell membranes, and it seems that IgD works as an antigen receptor. Recently, its relations to autoimmune diseases such as rheumatism, etc. have been also estimated. In this manner, IgD relates to essential mechanisms of immunity, and DNA coding for the H chain of IgD provided according to the present invention has a possibility for use as a diagnostic agent for diseases due to the abnormality of IgD.

Disclosure of the Invention

It is the best way to use a genetic engineering procedure in the mass production of human IgD, but no reports have been made about mRNA and cDNA coding for IgD. The present inventors have established a process for producing human IgD by isolating mRNA coding for human IgD from cells, introducing the mRNA into an in vitro protein synthesis system, thereby producing human IgD and recovering it, and have completed the present invention. Furthermore, they have synthesized cDNA coding for the H chain of human IgD, using the mRNA and determined a base sequence in the whole constant region and a portion of the variable region in the H chain of IgD, whereby it has been found that the amino acid sequence at C terminal of the H chain of human IgD is not the Met so far reported (Lin, et al.: Proc. Natl. Acad. Sci., U.S.A. 78 504 - 508, 1981), but "... Met Lys" having an additional Lys.

The present invention will be explained in detail below.

The present invention provides a process for producing human IgD using an isolated mRNA coding for human IgD. Furthermore, the present invention provides a process for producing whole or a portion of polypeptides of human IgD by synthesizing cDNA coding for IgD by use of the mRNA, introducing a recombinant harboring the cDNA into microorganisms, and culturing the microorganisms.

- 3 -

0146627

The processes for producing mRNA and cDNA are described below.

Cells secreting human IgD, for example, established myeloma cells, are cultured at 30 - 40°C for an appropriate time, for example, 20 - 150 hours, and then $10^{5-9}$ of the cells are subcutaneously injected into nude mice. The mice are bred for 2 - 3 weeks to subcutaneously form cancer cells. Then, 5 - 50 g of the cancer cells are collected and are ruptured, and after removal of nuclei, extracted with phenol to extract all RNA from the cytoplasm.

The extracted RNA is dissolved in a salt solution with a high salt concentration (for example, solutions of NaCl, KCl, etc.), and the solution is passed through a column of oligo (dT) cellulose (available from P-L Biochemicals, USA) to adsorb mRNA having poly A, and then the column is eluted with water or a solution with a low salt concentration (for example, 10 mM Tris-HCl buffer solution) to separate mRNA having poly A.

The separated mRNA is further fractionated mainly owing to differences in the molecular weight by sucrose density gradient centrifugation. IgD mRNA activity in the respective fractions is investigated in a system of reticulocyte lysate [H.R.B. Pelham, et al.: European J. Biochemistry 67 247 - 256 (1976)], and the presence of IgD in the synthesized protein is also investigated according to an immunological procedure by use of human IgD antibody [A.R. Midgley, et al.: Methods in Enzymology, Academic Press, 70 266 - 291 (1980)].

DNA complementary to mRNA is synthesized in vitro by a reverse transcriptase [obtainable from Avian myeloblastosis virus], using mRNA in the fraction of highest activity thus obtained as a temperate. The synthesis is carried out as follows.

mRNA is allowed to react with a reverse transcriptase at a specific temperature (for example, 37°C) for a specific time (for example, 60 minutes) together with oligo (dT) (available from P-L Biochemicals, USA), $MgCl_2$

(for example, 5 mM), NaCl (for example, 30 mM), mercapto-ethanol (for example, 5 mM), Tris-HCl buffer solution (for example, pH 8.0, 40 mM), etc., using deoxyadenosine triphosphate (dATP), deoxythymidine triphosphate (dTTP), deoxyguanosine triphosphate (dGTP), deoxycytidine triphos-phate (dCTP) (for example, 0.5 mM each) as substrates.

The thus obtained reaction product (DNA) is freed from protein by phenol, etc., and removed from the temperate RNA by alkali treatment and then synthesized again into double-stranded DNA with reverse transcriptase. The synthesis can be carried out in the same manner as in the aforegoing synthesis of DNA complementary to the mRNA except that DNA is used in place of mRNA and that oligo(dT) is not employed.

Similar double-stranded DNA can be synthesized by Escherichia coli DNA polymerase 1 (obtainable from Escherichia coli URE 600, etc.) in place of the reverse transcriptase.

The thus synthesized double-stranded DNA is treated with nuclease S 1 (obtainable from Aspergillus orizae) in the presence of $ZnCl_2$ (for example, 1 mM), sodium acetate buffer solution (for example, 0.1M, pH 4.5), NaCl (for example, 0.2M), etc., and then incubated at a specific temperature (for example, 37°C) for a specific time (for example, 20 minutes) in the presence of terminal transferase purified from calf thymus (available, for example, from Bethesda Research Laboratories, USA) and a potassium cacodylate buffer solution (for example, pH 7.6, 0.14M), Tris (base) (for example, 0.03M), dithiothreitol (for example, 0.1 mM), $CoCl_2$ (for example, 1 mM), dGTP (for example, 1 mM), etc., whereby deoxyguanine chains can be added to both 3' terminals of the synthesized DNA.

On the other hand, any vector as frequently used in Escherichia coli can be used. For example, pBR 322 or its derivatives are suitable. The synthesized double-stranded DNA can be inserted by using a unique restriction enzyme site existing in these vectors. For example, pBR 322 is treated with PstI in an appropriate buffer solution, for example, a buffer solution containing 6 mM Tris-HCl (pH 7.5), 6 mM MgCl$_2$, 6 mM NaCl and 6 mM 2-mercaptoethanol to cut at the unique PstI site in pBR 322 DNA and deoxycytidine chains are added to both 3' terminals of the cleaved DNA in the same manner as mentioned above except that dCTP is used in place of dGTP.

The thus obtained synthesized double-stranded DNA with the chains added to both 3' terminals and plasmid DNA are incubated at a specific temperature for a specific time together with, for example, Tris-HCl buffer solution (for example, pH 7.5, 50 mM), NaCl (for example, 0.1 M), EDTA (for example, 5 mM), etc. to hybridize the DNAs through hydrogen bonds due to guanine-cytosine, and then transformable Escherichia coli, for example, Escherichia coli χ1776 [Molecular Cloning of Recombinant DNA, Scott, W.A. & Werner, R. edited, Academic Press, 99 - 114 (1977)] (ATCC 31244) is transformed with the hybridized DNA by, for example, the procedure of Enea et al [J. Mol. Biol., 96, 475 - 509 (1975)].

The thus obtained novel recombinant plasmid contains tetracycline-resistant (Tc$^R$) genes derived from pBR 322, and so the transformed Escherichia coli shows Tc$^R$. Among the Tc$^R$ strains are screened strains containing a novel recombinant harboring a DNA complementary to the mRNA of IgD by positive hybridization translation assay [T. Taniguchi, et al.: Proc. Japan Acad. 55B 464 (1979); G.R. Stark, et al.: Nucleic Acids Res. 6 195 (1979)].

The assay is a procedure comprising hybridizing mRNA to cDNA in plasmid, then isolating the hybridized mRNA, and letting it translate to identify a product, more particularly a procedure which comprises digesting plasmid with a restriction enzyme, combining it with nitrocellulose or DBM-paper by denaturation, hybridizing the mRNA thereto, releasing the mRNA from the nitrocellulose filter, and letting it translate through a protein synthesis system of rabbit reticulocyte lysate.

Specifically, rabbit reticulocyte lysate (made by Amersham), mRNA and $^{35}$S-methionine are mixed with each other, and incubated at 25 - 37°C for 10 - 20 minutes, and 1 - 10 μl of the mixture is added to 0.5 ml of 1 M NaOH / $H_2O_2$ mixture. Then, the mixture is incubated at 30 - 40°C for 10 - 20 minutes, and aminoacyl-tRNA complex is hydrolyzed. With ice cooling, 2 ml of 25% trichloroacetic acid is added thereto, and the mixture is allowed to stand for at least 30 minutes. The product formed by the protein synthesis system is precipitated with an antibody to IgD (made by Hoechst) and subjected to electrophoresis with 10% SDS polyacrylamide gel electrophoresis to detect the product by autoradiography.

Strains that form products reacting with anti IgD antibody are identified according to this procedure. The procedure is also applicable to production of IgD by mRNA.

Another procedure for selecting the desired strain comprises synthesizing oligonucleotide (usually 14 - 18-mers) having a base sequence estimatable from the reported amino acid sequence of IgD and screening strains by using the oligonucleotide as a probe. The probe can be synthesized according to the ordinary triester procedure [J. Am. Chem. Soc., 97 7327 (1975)], and the screening procedure is carried out according to the procedure of Grunstein-Hogness [Proc. Natl. Acad. Sci., 72 3961 - 3965 (1975)].

mRNA coding for IgD according to the present invention is applicable to a process for producing IgD polypeptides by injecting the mRNA into oocytes of Xenopus laevis.

The cDNA (dsDNA) coding for IgD according to the present invention is applicable to quantitative determination of IgD, and a recombinant harboring cDNA (dsDNA) is applicable to mass production of human IgD by introducing it into Escherichia coli. Thus, they are very useful.

0146627

<u>Brief Description of Drawings</u>

Fig. 1 shows a restriction enzyme map of pIgD-1.

Fig. 2 shows an amino acid sequence of H chain in IgD and a base sequence coding for it.

<u>Best mode for carrying out the invention</u>

Examples of the present invention will be given below.

<u>Example 1</u>

Separation of mRNA coding for human IgD:

(1) Preparation of ODA cells secreting human IgD:

About $10^5$/ml established human myeloma cells ODA [cells obtained by treating myeloma cells from patients with human IgD myeloma according to a procedure for forming colonies in soft agar: Masaatsu Yamada, <u>et al.</u>: "Technique for Tissue Culture" compiled by Japan Tissue Culture Society and Published by Asakura Shoten 32 (1982)] was cultured at 37°C for 3 days in a medium prepared by adding 10% calf fetal serum (made by GIBCO) and 100 μg/ml penicillin to 500 ml of RPMI-1640 medium (made by GIBCO) in a roller bottle. Then, about $10^7$ of ODA cells recovered from the culture liquor was irradiated with $^{60}$Co 500 red and subcutaneously injected into about 20 nude mice (obtained from Nippon Rat) two days thereafter. About 1 g of cancer cells were formed in about 3 weeks.

(2) Isolation of mRNA coding for human IgD:

About 30 g of cancer cells subcutaneously formed in the said nude mice were recovered, dispersed in 80 ml of a buffer solution [0.88 M sucrose, 25 mM KCl, 50 mM Tris-HCl (pH 7.5), 5 mM $MgCl_2$] and ground six times in a teflon homogenizer. The resulting solution was laid as a layer on 8 ml of 1.75 M sucrose and 8 ml of 1.5 M sucrose in a centrifuge tube for Spinco SW 27 rotor (made by Beckmann) and centrifuged at 2,500 rpm for one hour to fractionate polysomes containing RNA.

Then, 20 ml of a mixture of 0.1 M NaCl and 0.1 M Tris-HCl (pH 9.0) was added to 20 ml of the thus obtained membrane-bound type polysome fractions, and further 40 ml of a mixture of phenol : chloroform : isoamyl alcohol (50 : 50 : 1) was added thereto. The mixture was shaked to extract RNA. Sodium chloride was added to 35 ml of the RNA extract to make 0.2 M sodium chloride, and cooled ethanol was further added thereto to make a final ethanol concentration of 70% to precipitate RNA at -20°C.

Then, 25 mg of the thus obtained precipitates were dissolved in 25 ml of a solution of 10 mM Tris-HCl (pH 7.5), 0.5 M NaCl, 1 mM EDTA and 0.1% sodium dodecyl sulfate (referred to as "SDS" hereinafter), and the resulting solution was passed through 1.5 ml of oligo (dT) cellulose column (made by P-L Biochemicals).

Adsorbed mRNA containing poly A was eluted with a salt solution of low concentration [10 mM Tris-HCl (pH 7.5)] and water alternately to separate 350 µg of mRNA containing poly A.

(3)　Fractionation of mRNA by sucrose density gradient centrifugation:

The thus obtained mRNA was precipitated with ethanol, and the precipitates were dissolved in 0.5 ml of 1 mM EDTA. The solution was heated at 70°C for 2 minutes, and centrifuged at 40,000 rpm at 15°C for 15 hours in direct density gradients of 5 - 25% sucrose prepared in a solution containing 10 mM Tris-HCl, 0.1% SDS and 1 mM EDTA by Beckmann SW40 rotor (made by Beckmann).

To make a size marker, ribosomal RNA was centrifuged in another centrifuge tube simultaneously.

The said centrifuge fractions were sampled in 330 fractions and a portion of each fraction was investigated for the formation of IgD in a rabbit reticulocyte system, as shown in Example 2. As a result, IgD mRNA was found in fractions (22 - 24) corresponding to sedimentation coefficient of about 17S.

Example 2

Production of human IgD:

At first, 11 μl of a solution containing 8 μl of rabbit reticulocyte lysate (made by Amarsham), $^{35}$S-methionine (0.38 μCi/2 μl and 1 μl of mRNA (0.5 μg) was incubated at 30°C for 60 minutes. Then, 5 μl of commercially available IgD antibody (made by Hoechst) was added to 5 μl of the reaction mixture, and the solution was incubated at 37°C for 2 hours, and centrifuged at 12,000 rpm for 20 minutes. Precipitates were collected, and it was confirmed by autoradiography that products with the same mobility as that of IgD standard substance were obtained in 10% SDS-polyacrylamide gel electrophoresis.

Production of IgD free of isotope was also confirmed by Coomassie Blue staining after the same reaction as mentioned above except that $^{35}$S-methionine is removed from the system and gel electrophoresis.


Example 3

Preparation of cDNA coding for the H chain of human IgD:

At first, 10 μg of human IgD mRNA fractions obtained in Example 1 was incubated at 42°C for 2 hours in 200 μl of reaction solution [0.5 mM each of dATP, dGTP, dTTP and dCTP, 1 μg of oligo (dT), 50 units of AMV reverse transcriptase (made by Life Science), 10 mM $MgCl_2$, 50 mM KCl, and 50 mM Tris-HCl (pH 8.3)] to synthesize cDNA complementary to human IgD mRNA.

Protein was removed therefrom with phenol, and RNA was removed therefrom by treatment with 0.3N NaOH at 60°C for 30 minutes.

Then, 2.5-fold volume of ethanol was added thereto to precipitate about 1.5 μg of cDNA.

The cDNA was transcribed with an reverse transcriptase in the same manner as above, and single chain portions were removed therefrom with 2.5 units of S1 nuclease (made by Bethesda Research Laboratories) to obtain 0.45 μg of double stranded DNA (dsDNA).

0146627

Then, 0.3 µg of dsDNA was dissolved in 50 µl of a reaction solution [0.14 M potassium cacodylate (pH 7.6), 0.03 M Tris-HCl (pH 7.6), 0.1 mM dithiothreitol, 1 mM CoCl₂ and 1 mM dCTP], admixed with 10 units of terminal transferase (made by BRL), and incubated at 37°C for 15 minutes, whereby about 31 deoxycytidine chains were added to both 3' terminals of the dsDNA.

Example 4

Cloning of ds-DNA coding for the H chain of human IgD and determination of base sequence:

At first, 8 µg of plasmid pBR 322 was dissolved in 100 µl of a buffer solution [6 mM Tris-HCl (pH 7.5), 6 mM MgCl₂, 6 mM NaCl, and 6 mM 2-mercaptoethanol], admixed with 16 units of PstI (made by Takara Shuzo), and incubated at 37°C for 2 hours to cleave pBR 322.

DNA of the cleaved pBR 322 was recovered by precipitation with ethanol, and dissolved in 100 µl of a solution containing 0.14 M potassium cacodylate, 30 mM Tris-HCl (pH 7.6), 1 mM dGTP, 0.1 mM dithiothreitol, and 1 mM CoCl₂, admixed with 10 units of terminal transferase (made by Bethesda Research Laboratories), and incubated at 37°C for 30 minutes, whereby about 30 deoxyguanine chains were added to the 3' terminal of the cleaved pBR 322 DNA.

Then, 50 ng of the thus obtained pBR 322 DNA and 10 ng of the ds-DNA were incubated in 50 µl of a solution comprising 20 mM Tris-HCl (pH 7.5), 100 mM NaCl and 1 mM EDTA at 65°C for 2 minutes, 46°C for 120 minutes, 37°C for 60 minutes and room temperature for 60 minutes to hybridize both DNAs.

With this reaction solution, Escherichia coli χ1776 (ATCC 31244) was transformed. About 3,000 tetracycline-resistant strains (Tc$^R$) were separated, and 2,000 strains were selected among them, and clones forming proteins crossing with anti IgD antibody were selected by said positive hybridization translation assay.

A plasmid was obtained from one strain selected according to the ordinary procedure [H.C. Birnboim, et al.:

0146627

Nucleic Acids Research 7 1513 (1979)] and named pIgD-1.

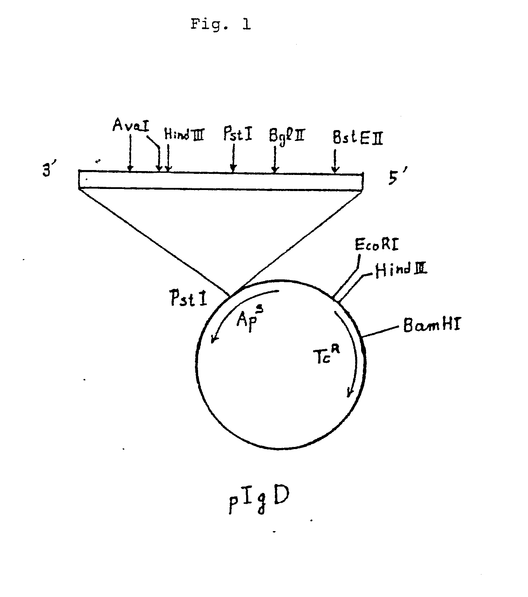

The pIgD-1 plasmid was digested with PstI, EcoRI, HindIII, BstEII, BglII and AvaI,. and then subjected to agarose gel electrophoresis. As a result, it was confirmed that pIgD-1 had the structure of Fig. 1.

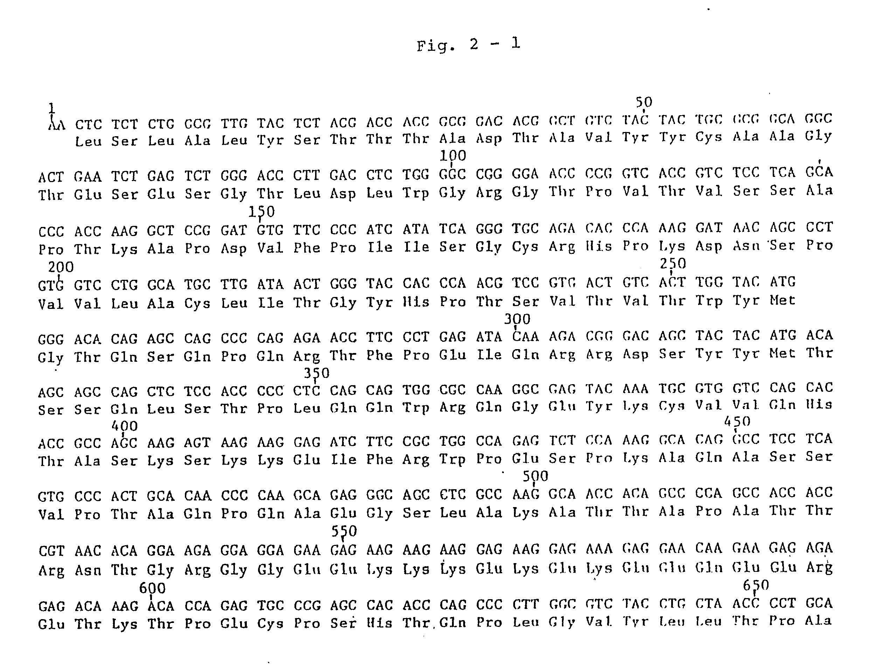

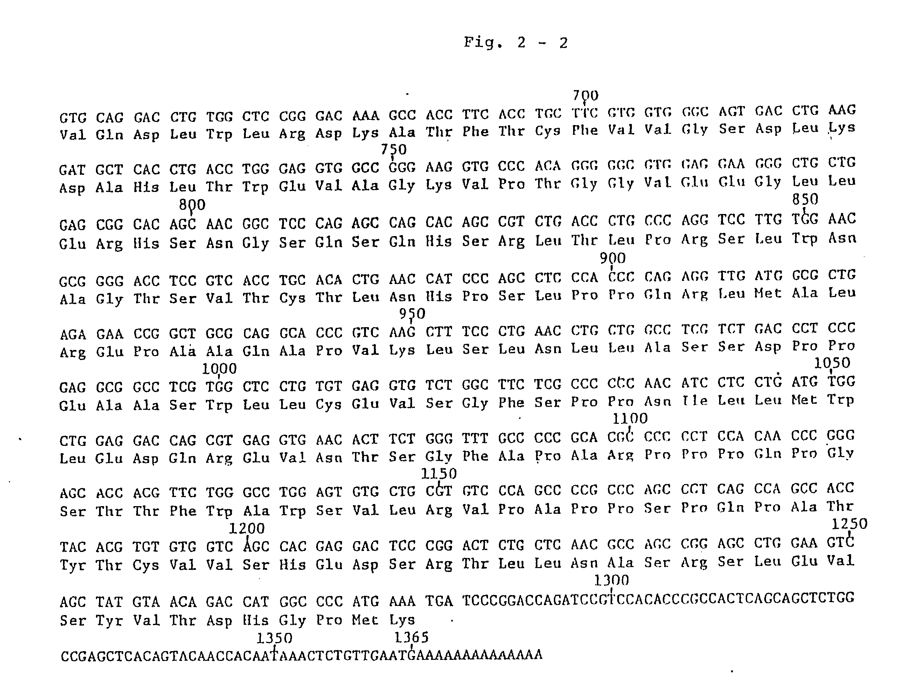

It was confirmed by determination of base sequences of various DNA fragments separated from pIgD-1, for example, PstI fragment and HindIII fragment according to the procedure of Maxam-Gilbert [Proc. Natl. Acad. Sci., 74 560 - 564 (1977)] that a gene coding for the H chain of IgD existed at the portions of about 1.4 Kb inserted in the present plasmid. The result is shown in Fig. 2.

The sequence from Ala (alanine) at the 43rd position to Met (methionine) at the 416th position in Fig. 2 was in complete accordance with the known amino acid sequence in the constant region in the H chain of human IgD. It was confirmed for the first time that Lys (lysine) was present at the C terminal of the H chain.

Escherichia coli strain containing IgD-1 was deposited with Fermentation Research Institute of Agency of Industrial Science and Technology, Ibaraki, Japan as Escherichia coli NIgD-1 under FERM P-7028 on April 2, 1983 and converted into the international deposition under the Budapest Treaty as FERM BP-339 on August 24, 1983.

BAD ORIGINAL

0146627

Claims

1. A messenger RNA (referred to as "mRNA" hereinafter) coding for human immunoglobulin D (referred to as "IgD" hereinafter) separated from human cells.

2. The mRNA according to claim 1, characterized in that the cells are human myeloma cells.

3. The mRNA according to claim 1, characterized in that the amino acid sequence of the H chain in the IgD is shown in Fig. 2.

4. A process for producing mRNA, characterized by culturing cells of capable of producing an IgD in a medium, thereby forming and accumulating mRNA coding for IgD in the culture broth, and recovering it therefrom.

5. A DNA complementary to mRNA of IgD.

6. The DNA according to claim 5, characterized in that the base sequence corresponding to the H chain in the IgD is shown in Fig. 2.

7. A recombinant DNA of a DNA complementary to mRNA of IgD and a vector DNA.

8. The recombinant DNA according to claim 7, characterized by pIgD-1.

9. A microorganism containing a recombinant DNA of a DNA complementary to mRNA of IgD and a vector DNA.

10. The microorganism according to claim 9, characterized by Escherichia coli NIgD-1.

11. A process for producing IgD, characterized by introducing an mRNA coding for IgD into an in vitro protein synthesis system, thereby forming IgD, and recovering it.

12. The process according to claim 11, characterized in that the in vitro protein synthesis system is a rabbit reticulocyte system or an oocyte system of Xenopus laevis.

13. The process according to claim 11, characterized in that the amino acid sequence of the H chain in the IgD is shown in Fig. 2.

14. A process for producing IgD, characterized by culturing a microorganism containing a recombinant DNA of a DNA complementary to mRNA of IgD and a vector DNA in a culture medium, thereby forming and accumulating IgD in the culture broth, and recovering the IgD therefrom.

15. The process according to claim 14, characterized in that the amino acid sequence of the H chain in the IgD is shown in Fig. 2.

1/3

Fig. 1

pIgD

Fig. 2 - 1

AA CTC TCT CTG GCG TTG TAC TCT ACG ACC ACC GCG GAC ACG GCT GTC TAC TAC TGC GCG GCA GGC
   Leu Ser Leu Ala Leu Tyr Ser Thr Thr Thr Ala Asp Thr Ala Val Tyr Tyr Cys Ala Ala Gly

ACT GAA TCT GAG TCT GGG ACC CTT GAC CTC TGG GGC CGG GGA ACC CCG GTC ACC GTC TCC TCA GCA
Thr Glu Ser Glu Ser Gly Thr Leu Asp Leu Trp Gly Arg Gly Thr Pro Val Thr Val Ser Ser Ala

CCC ACC AAG GCT CCG GAT GTG TTC CCC ATC ATA TCA GGG TGC AGA CAC CCA AAG GAT AAC AGC CCT
Pro Thr Lys Ala Pro Asp Val Phe Pro Ile Ile Ser Gly Cys Arg His Pro Lys Asp Asn Ser Pro

GTG GTC CTG GCA TGC TTG ATA ACT GGG TAC CAC CCA ACG TCC GTG ACT GTC ACT TGG TAC ATG
Val Val Leu Ala Cys Leu Ile Thr Gly Tyr His Pro Thr Ser Val Thr Val Thr Trp Tyr Met

GGG ACA CAG AGC CAG CCC CAG AGA ACC TTC CCT GAG ATA CAA AGA CGG GAC AGC TAC TAC ATG ACA
Gly Thr Gln Ser Gln Pro Gln Arg Thr Phe Pro Glu Ile Gln Arg Arg Asp Ser Tyr Tyr Met Thr

AGC AGC CAG CTC TCC ACC CCC CTC CAG CAG TGG CGC CAA GGC GAG TAC AAA TGC GTG GTC CAG CAC
Ser Ser Gln Leu Ser Thr Pro Leu Gln Gln Trp Arg Gln Gly Glu Tyr Lys Cys Val Val Gln His

ACC GCC AGC AAG AGT AAG AAG GAG ATC TTC CGC TGG CCA GAG TCT CCA AAG GCA CAG GCC TCC TCA
Thr Ala Ser Lys Ser Lys Lys Glu Ile Phe Arg Trp Pro Glu Ser Pro Lys Ala Gln Ala Ser Ser

GTG CCC ACT GCA CAA CCC CAA GCA GAG GGC AGC CTC GCC AAG GCA ACC ACA GCC CCA GCC ACC ACC
Val Pro Thr Ala Gln Pro Gln Ala Glu Gly Ser Leu Ala Lys Ala Thr Thr Ala Pro Ala Thr Thr

CGT AAC ACA GGA AGA GGA GGA GAA GAG AAG AAG AAG GAG AAG GAG AAA GAG GAA CAA GAA GAG AGA
Arg Asn Thr Gly Arg Gly Gly Glu Glu Lys Lys Lys Glu Lys Glu Lys Glu Glu Gln Glu Glu Arg

GAG ACA AAG ACA CCA GAG TGC CCG AGC CAC ACC CAG CCC CTT GGC GTC TAC CTG CTA ACC CCT GCA
Glu Thr Lys Thr Pro Glu Cys Pro Ser His Thr Gln Pro Leu Gly Val Tyr Leu Leu Thr Pro Ala

Fig. 2 - 2

```
                                                                    700
GTG CAG GAC CTG TGG CTC CGG GAC AAA GCC ACC TTC ACC TGC TTC GTG GTG GGC AGT GAC CTG AAG
Val Gln Asp Leu Trp Leu Arg Asp Lys Ala Thr Phe Thr Cys Phe Val Val Gly Ser Asp Leu Lys
                                                750
GAT GCT CAC CTG ACC TGG GAG GTG GCC GGG AAG GTG CCC ACA GGG GGC GTG GAG GAA GGG CTG CTG
Asp Ala His Leu Thr Trp Glu Val Ala Gly Lys Val Pro Thr Gly Gly Val Glu Glu Gly Leu Leu
         800                                                                         850
GAG CGG CAC AGC AAC GGC TCC CAG AGC CAG CAC AGC CGT CTG ACC CTG CCC AGG TCC TTG TGG AAC
Glu Arg His Ser Asn Gly Ser Gln Ser Gln His Ser Arg Leu Thr Leu Pro Arg Ser Leu Trp Asn
                                                            900
GCG GGG ACC TCC GTC ACC TGC ACA CTG AAC CAT CCC AGC CTC CCA CCC CAG AGG TTG ATG GCG CTG
Ala Gly Thr Ser Val Thr Cys Thr Leu Asn His Pro Ser Leu Pro Pro Gln Arg Leu Met Ala Leu
                                     950
AGA GAA CCG GCT GCG CAG GCA CCC GTC AAG CTT TCC CTG AAC CTG CTG GCC TCG TCT GAC CCT CCC
Arg Glu Pro Ala Ala Gln Ala Pro Val Lys Leu Ser Leu Asn Leu Leu Ala Ser Ser Asp Pro Pro
         1000                                                                       1050
GAG GCG GCC TCG TGG CTC CTG TGT GAG GTG TCT GGC TTC TCG CCC CCC AAC ATC CTC CTG ATG TGG
Glu Ala Ala Ser Trp Leu Leu Cys Glu Val Ser Gly Phe Ser Pro Pro Asn Ile Leu Leu Met Trp
                                                            1100
CTG GAG GAC CAG CGT GAG GTG AAC ACT TCT GGG TTT GCC CCC GCA CGC CCC CCT CCA CAA CCC GGG
Leu Glu Asp Gln Arg Glu Val Asn Thr Ser Gly Phe Ala Pro Ala Arg Pro Pro Pro Gln Pro Gly
                                     1150
AGC ACC ACG TTC TGG GCC TGG AGT GTG CTG CGT GTC CCA GCC CCG CCC AGC CCT CAG CCA GCC ACC
Ser Thr Thr Phe Trp Ala Trp Ser Val Leu Arg Val Pro Ala Pro Pro Ser Pro Gln Pro Ala Thr
         1200                                                                       1250
TAC ACG TGT GTG GTC AGC CAC GAG GAC TCC CGG ACT CTG CTC AAC GCC AGC CGG AGC CTG GAA GTC
Tyr Thr Cys Val Val Ser His Glu Asp Ser Arg Thr Leu Leu Asn Ala Ser Arg Ser Leu Glu Val
                                                            1300
AGC TAT GTA ACA GAC CAT GGC CCC ATG AAA TGA TCCCGGACCAGATCCGTCCACACCCGCCACTCAGCAGCTCTGG
Ser Tyr Val Thr Asp His Gly Pro Met Lys
                     1350              1365
CCGAGCTCACAGTACAACCACAATAAACTCTGTTGAATGAAAAAAAAAAAAAA
```

0146627

## I. CLASSIFICATION OF SUBJECT MATTER (if several classification symbols apply, indicate all) [3]

According to International Patent Classification (IPC) or to both National Classification and IPC

Int. Cl.[3] C12N 15/00, C07H 21/00, C12P 19/34, C12N 1/20, C12P 21/00 // A61K 37/04, (C12N 1/20, C12R 1/19)

## II. FIELDS SEARCHED

Minimum Documentation Searched [4]

| Classification System | Classification Symbols |
|---|---|
| I P C | C12N 15/00, C07H 21/00, C12P 19/34, C12N 1/20, C12P 21/00, A61K 37/04 |

Documentation Searched other than Minimum Documentation
to the Extent that such Documents are Included in the Fields Searched [5]

## III. DOCUMENTS CONSIDERED TO BE RELEVANT [14]

| Category* | Citation of Document, [16] with indication, where appropriate, of the relevant passages [17] | Relevant to Claim No [18] |
|---|---|---|
| E | JP,A, 58-21963 (Takeda Chemical Industries, Ltd.) 8. February. 1983 (08. 02. 83) | 1 - 15 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search [2] | Date of Mailing of this International Search Report [2] |
|---|---|
| November 14, 1983 (14. 11. 83) | November 28, 1983 (28. 11. 83) |

| International Searching Authority [1] | Signature of Authorized Officer [20] |
|---|---|
| Japanese Patent Office | |

Form PCT/ISA/210 (second sheet) (October 1981)